# EUROPÄISCHE PATENTSCHRIFT

(11) **EP 3 218 353 B1**
(45) Veröffentlichungstag und Bekanntmachung des Hinweises auf die Patenterteilung: **19.08.2020**
(21) Anmeldenummer: 15790990.4
(22) Anmeldetag: 09.11.2015
(51) Int. Cl.: C07C 231/02

(54) **KONTINUIERLICHER PROZESS ZUR HERSTELLUNG EINES TENSIDS IN EINEM ROHRREAKTOR**
CONTINUOUS PROCESS FOR PRODUCING A TENSIDE IN A TUBE REACTOR
PROCESSUS CONTINU DE PRODUCTION D'UN TENSIOACTIF DANS UN RÉACTEUR TUBULAIRE

(30) Priorität: 13.11.2014 DE 102014016841; 11.05.2015 DE 102015006119
(43) Veröffentlichungstag der Anmeldung: 20.09.2017
(73) Patentinhaber: Clariant International Ltd, 4132 Muttenz (CH)
(72) Erfinder: APPEL, Jörg, 84577 Tüßling (DE); HEITMANN, Dennis, 83355 Grabenstätt (DE); WERNER, Sarah, 84453 Mühldorf (DE)
(74) Vertreter: Mikulecky, Klaus
(86) Internationale Anmeldenummer: PCT/EP2015/076072
(87) Internationale Veröffentlichungsnummer: WO 2016/075080

(56) Entgegenhaltungen:
- EP-A2- 0 633 244

## Beschreibung

Die folgende Erfindung bezieht sich auf einen kontinuierlichen Prozess zur Herstellung von Tensiden, insbesondere von Polyhydroxyfettsäureamiden (z. B. N-Methylglucamide) in einem Rohrreaktor. Tenside können zum Beispiel als oberflächenaktive Substanzen, beispielsweise in Waschmittelformulierungen, eingesetzt werden. Das erfindungsgemäße Verfahren erlaubt es Tenside kontinuierlich mit hoher Ausbeute und Reinheit zu produzieren.

### Hintergründe zur Erfindung

Für den Einsatz In Waschmittelformulierungen, personal care Produkten und im Pflanzenschutz existiert eine große Vielzahl an nichtionischen Tensiden. Eine Alternative zu vielen herkömmlichen Tensiden sind Polyhydroxyfettsäureamide, welche wertvolle und vielfach einsetzbare oberflächenaktive Verbindungen sind.

So können sie zum Beispiel als solche oder in Mischung mit anionischen, kationischen und/oder nichtionischen Tensiden als Reinigungsmittel, Waschmittel, Textilbehandlungsmittel oder dergleichen eingesetzt werden und zwar in Form von Festprodukten (zum Beispiel als Pulver, Körner, Granulat oder Schuppen), Lösungen, Dispersionen, Emulsionen Pasten und dergleichen. Da Polyhydroxyfettsäureamide auch gut biologisch abbaubar sind und aus nachwachsenden Rohstoffen hergestellt werden können, haben sie in jüngster Zeit größere Bedeutung erlangt.

Dementsprechend würde die Vermutung nahe liegen, dass diese Verbindungen ihre Anwendung in einer Vielzahl von Formulierungen gefunden haben. Diese Vermutung kann aber nicht bestätigt werden. Als Grund dafür, wird davon ausgegangen, dass die Herstellung dieser Polyhydroxyfettsäureamide vor allem in der gewünschten Qualität und Reinheit zu anspruchsvoll ist. Die Herstellung von Polyhydroxyfettsäureamiden mit Batchverfahren wurde zwar zahlreich beschrieben, allerdings resultieren diese Verfahren in einer nicht zufriedenstellenden Qualität. Vor allem für den Einsatz im personal care Bereich ist eine strenge Spezifikation in Bezug auf z. B. Farbe, Lösemittelgehalt und Nebenkomponenten einzuhalten. Dies ist mit den in der Literatur beschriebenen Verfahren nicht auf einem ökonomischen Weg möglich. Insbesondere können hohe Verweilzeiten mit starker Temperaturbelastung, schlechte Vermischung und starkes Schäumen in einem absatzweise betriebenen Verfahren zu erheblichen Problemen führen.

Die Herstellung in einem Batchverfahren erlaubt es, gezielt auf die verschiedenen Anforderungen während der Reaktion einzugehen. So können verschiedene rheologische Phasen auftreten, die je nach Produkt unterschiedlich lange Bestand haben. Ein kontinuierliches Verfahren muss diese Herausforderung meistern und dies ist ein Grund, warum bisher keine kontinuierlichen Verfahren beschrieben wurden, die die genannten Qualitätsanforderungen erreichen.

EP 0633244 A2 beschreibt den Betrieb einer Rührkesselkaskade zur Herstellung von Polyhydroxyfettsäureamiden. Dieser Prozess hat jedoch den Nachteil einer breiten Verweilzeitverteilung, welche vermehrt zur Bildung von unterwünschten Nebenprodukten führt. Auf Grund der hohen Reaktionsvolumina und damit verbundener ungünstiger Temperaturverteilung im Reaktionssystem ist die thermische Belastung zu hoch, was sich negativ auf die Farbe der Produkte auswirkt.
Des Weiteren hat dieser Prozess den Nachteil, dass es zu einer starken Schaumbildung bei der Entfernung der Koppelprodukte kommt. Die Schaumbildung führt dazu, dass der erwünschte geringe Druck nicht erreicht werden kann um die Koppelprodukte komplett zu entfernen. Um die Entfernung der Koppelprodukte sicherzustellen müsste eine höhere Temperatur beziehungsweise ein geringerer Durchsatz verwendet werden, was zu einer höheren Temperaturbelastung des Endproduktes führt. Eine hohe Temperaturbelastung führt allerdings zu einem höheren Gehalt an Nebenkomponenten und einer schlechteren Farbe.

Um die oben genannten Probleme zu überwinden wurde ein neues, kontinuierliches Verfahren entwickelt bei dem durch verkürzte Verweilzeit und optimierte Temperaturkontrolle Polyhydroxyfettsäureamide mit verbesserter Qualität und in größeren Ausbeuten hergestellt werden können. Die verbesserte Qualität zeigt sich insbesondere in einer Verringerung farbgebender Substanzen und der Reduktion von Nebenkomponenten (GFZ <3, cyclisches <0,1 %). Des Weiteren ist es mit dem neuen Verfahren möglich einen MeOH-Gehalt von <0,3 % im Endprodukt zu gewährleisten.

### Beschreibung der Erfindung

Überraschend wurde nun gefunden, dass die Herstellung von Tensiden, insbesondere von Polyhydroxyfettsäureamiden mit dem erfindungsgemäßen kontinuierlichen Verfahren ökonomischer und mit höherer Produktqualität möglich ist. Da das Produkt sehr temperatursensitiv ist, vor allem was den Gehalt an cyclischen Amiden und die Farbe des Produktes betrifft, ist ein kontinuierlicher Herstellprozess in einem Rohrreaktor mit geringer Verweilzeit eine wesentlich bessere Alternative, um eine sehr gute Produktqualität zu erreichen. Des Weiteren können dadurch die Produktionskosten gesenkt werden.

Die vorliegende Erfindung befasst sich mit einem kontinuierlichen Prozess zur Herstellung eines Tensides, enthaltend eine Verbindung der Formel (1), wobei R² ein Fettsäurealkylrest ist und R¹ ein geradkettiger (linearer) oder verzweigter C₁ bis C₁₂, vorzugsweise ein C₁ bis C₈, insbesondere ein C₁-C₆ Kohlenwasserstoffrest, beispielsweise einen Methyl, Ethyl, Propanyl, Isopropyl, n-Butanyl, iso-Butyl Rest darstellt. Insbesondere bevorzugt sind Methyl und Ethyl; im speziellen Methyl. X ist im Bereich von 1 bis 15, insbesondere 2 bis 6.

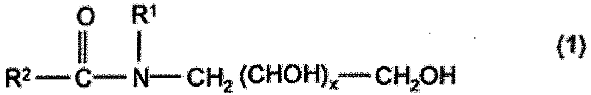

Die Herstellung des Tensides erfolgt dabei durch Umsetzung von Fettsäurealkylestern oder Fettsäuretriglyceriden mit einer N- n-alkylierten Polyhydroxyverbindung in Anwesenheit eines Alkalikatalysators oder eines Katalysators ausgewählt aus Hydroxiden oder Alkoholaten der 2. und 4. Nebengruppe des Periodensystems bei einer Temperatur im Bereich von 40 bis 300 °C, wobei das Verfahren die folgenden Schritte umfasst:
1. Mischen der wässrigen Rohstofflösung enthaltend eines oder mehrere N-Alkylglucamine mit dem Katalysator;
2. Trocknung der wässrigen Lösung, wobei der Wassergehalt nach der Trocknung im Bereich von 0 - 5 Gew.-%, liegt;
3. Erhitzen der getrockneten Mischung auf eine Temperatur im Bereich von 60 bis 180 °C und Mischen der Schmelze mit Fettsäurealkylestern oder Triglyceriden;
4. gegebenenfalls Zwischenspeichern und Mischen des Reaktionsgemisches in einem kontinuierlich betriebenen Rührkessel;
5. Reaktion im Rohrreaktor; und
6. gegebenenfalls Entfernung der Koppelprodukte.

Der Anteil der Verbindung (1) im Tensid liegt üblicherweise im Bereich von 15 bis 100 Gew.-%, vorzugsweise im Bereich von 30 bis 100 Gew.-%

Der Prozess beinhaltet die Reaktion von Fettsäurealkylestern oder Fettsäuretriglyceriden der Kettenlängen C₄ bis C₅₀ bevorzugt C₆ bis C₂₈, insbesondere C₆ bis C₂₀ und N-Alkylglucaminen in Anwesenheit von Alkalikatalysatoren, bevorzugt Alkalihydroxide und Alkalialkoholate insbesondere Natriumhydroxid, Natriummethanolat, Natriumethanolat, Kaliumhydroxid oder Kaliummethanolat, Kaliumethanolat als Katalysator in einem kontinuierlichen Rohrreaktor. Weiterhin lassen sich als Katalysatoren Hydroxide oder Alkoholate der 2. und 4. Nebengruppe des Periodensystems verwenden, wie beispielsweise Titan-tetra-isopropylat.

Der erfindungsgemäße Prozess setzt N-Alkylglucamine, z. B. N-Methyl D-Glucamine zu linearen Glucamidtensiden von sehr hoher Qualität um, welche auch ohne einen weiteren Aufbereitungsschritt (z. B. Umkristallisation) für die Anwendung im personal-care Bereich geeignet sind.

Das erfindungsgemäße Verfahren beinhaltet 4 bis 7 Prozessschritte:
1. Mischen der wässrige N-Alkylglucamin Rohstofflösung (Konzentration 30 - 90 Gew.-%, Amin in Wasser) mit 0,01 - 5 Gew.-% vorzugsweise 0,05 - 4 Gew.-%, insbesondere 1 - 3 Gew.-% Katalysator, wobei das Mischen in beliebiger Reihenfolge möglich ist.
   Die Temperatur sollte beim Mischen 40- 120 °C, vorzugsweise 50- 100 °C, insbesondere 50 - 90 °C betragen, wobei das Mischen bei einem Druck von 0 - 6 bar, normalerweise aber bei Normaldruck erfolgt.
2. Trocknung der wässrigen Lösung: Die Trocknung sollte möglichst effektiv sein und einen Wassergehalt nach der Trocknung von 0 - 5 % Gew.-%, vorzugsweise 0 - 3 Gew.-% und insbesondere 0 -1 Gew.-% nicht überschreiten.
   Die Temperatur beim Trocknen (2) beträgt 80- 180 °C; vorzugsweise 90 - 160 °C; insbesondere 100 - 150 °C, wobei sowohl unter Normaldruck als auch im Vakuum gearbeitet werden kann. Üblicherweise liegt der Druck bei diesem Schritt im Bereich von 0 - 2 bar, vorzugsweise 0 -1 bar; insbesondere 0,01 - 1 bar.
3. Erhitzen der getrockneten N-Alkylglucamin Mischung auf eine Temperatur im Bereich von 60- 180 °C, vorzugsweise 70- 160 °C; insbesondere 80 - 150 °C, bei einem Druck im Bereich von 0 - 6 bar und mischen der N-Alkylglucamin-Schmelze mit Fettsäurealkylestern im molaren Verhältnis N-Alkylglucamin-Schmelze : Fettsäurealkylester von 1:0,85 bis 1:1,2 , bevorzugt 1:0,9 bis 1,15, besonders bevorzugt 1:0.95 bis 1:1,1 bzw. bei Verwendung von Triglyceriden im molaren Verhältnis N-Alkylglycamin-Schmelze : Triglycerin von 0,25:1 bis 0,45:1, vorzugsweise von 0,3:1 bis 0,4:1, insbesondere von 0,33:1 bis 0,37:1. Die mittlere Verweilzeit liegt dabei üblicherweise im Bereich von 1 Minute bis 2 Stunden; vorzugsweise 2 min bis 1 h; insbesondere bei 5 - 45 Minuten.
4. (optional) Zwischenspeichern und Mischen des Reaktionsgemisches in einem kontinuierlich betriebenen Rührkessel. Hierbei liegt die Temperatur im Rührkessel üblicherweise bei 60 - 300 °C; vorzugsweise 70 - 200 °C; insbesondere bei 80 - 150 °C, bei Drücken von 0 - 200 bar, vorzugsweise 0,5 - 80 bar, insbesondere bei 1 - 5 bar.
5. Die Reaktion im Rohrreaktor erfolgt bei einer Temperatur im Bereich von 60 - 300 °C; vorzugsweise 70 - 200 °C; insbesondere bei 80 - 150 °C, bei Drücken von 0 - 200 bar, vorzugsweise 0,5 - 80 bar, insbesondere bei 1 - 20 bar. Die mittlere Verweilzeit liegt dabei üblicherweise im Bereich von 2 Minuten bis 2 Stunden; vorzugsweise bei 2 Minuten bis 1 Stunde; insbesondere bei 5 - 45 Minuten und gegebenenfalls
6. Entfernung der Koppelprodukte. Die Entfernung der Koppelprodukte ist optional und erfolgt bei einer Temperatur im Bereich von 50 - 180 °C; vorzugsweise bei 60 - 160 °C; insbesondere bei 70 - 150 °C. Der applizierte Druck kann dabei von Normaldruck bis Vakuum reichen und liegt üblicherweise bei 0 - 2 bar, vorzugsweise bei 0 -1 bar; insbesondere bei 0,01 - 1 bar. Üblicherweise erfolgt die Entfernung der Koppelprodukte durch dem Fachmann bekannte Verfahren, wie z. B. durch kontinuierliche Destillation oder mit Hilfe eines Dünnschichtverdampfers (auch Kurzwegverdampfer oder Fallfilmverdampfer).
7. (optional) Formulierung des Produktes:
   Zur einfacheren Handhabung in anschließenden Verarbeitungsschritten können die laut erfindungsgemäßem Prozess erhaltenen Produkte der Formel (1) mit verschiedenen Lösungsmitteln wie Wasser, Alkoholen (z. B. Ethanol, Isopropanol, Cetearylalkohol), Glykolen (Ethylenglykol, Propylenglykol, Polypropylenglykole, Polyethylenglykole) und deren Mischungen sowie mit Zusätzen wie Konservierungsstoffe (z. B. Methylisothiazolinon; Benzoesäure, Sorbinsäure) und Stoffe zur Einstellung des pH-Wertes (z. B. Zitronensäure, Milchsäure, Benzoesäure und weitere) abgemischt (formuliert) werden. Dies erfolgt üblicherweise bei Temperaturen im Bereich von 10 bis 130 °C; vorzugsweise bei 20 bis 110 °C unter Normaldruck.

Es hat sich gezeigt, dass in den jeweiligen Reaktoren eine Temperaturdifferenz zwischen der Reaktorwand und der Kernströmung von < 20 °C vorliegt. Vorzugsweise sollte diese Temperaturdifferenz < 10 °C, insbesondere < 5 °C betragen, um die gewünschte hohe Qualität und Reinheit der Produkte zu gewähren.

Der bei der Reaktion entstehende Alkohol kann über einen kontinuierlich betriebenen Dünnschichtverdampfer oder ähnliche Vorrichtungen, die dem Fachmann bekannt sind, abgetrennt werden. Einer der Vorteile des erfindungsgemäßen Verfahrens ist, dass es während des gesamten Prozesses zu keiner bzw. sehr geringer Schaumbildung kommt. Bei Reaktionen, bei welchen N-Alkylglucamin mit Triglyceriden umgesetzt wird, ist der letzte Reaktionsschritt nicht notwendig. Hier entsteht Glycerin als Koppelprodukt welches im Endprodukt verbleiben kann. Falls die N-Alkylglucamine als wässrige Lösung vorliegen, wird mit Hilfe eines kontinuierlichen Trocknungsverfahrens (z. B. Dünnschichtverdampfer) eine Schmelze hergestellt.

Im Mischer muss bauartbedingt eine hohe Phasengrenzfläche ermöglicht werden und/oder durch die Zugabe kleiner Mengen (0 - 10 %) von Phasentransferstoffen ein guter Phasenaustausch gewährleistet werden. Die Phasentransferstoffe können erfindungsgemäß in den ersten drei Schritten, also 1, 2 oder 3, zugesetzt werden. Als Phasentransferstoffe sind zum Beispiel Wasser und/oder Alkohole (Ethanol, Methanol, Propanol, Iso-Propanol, Butanol, Iso-Butanol), Glykole (Propylenglykol, Monoethylenglykol), N-Alkylglucamide, Polyalkylglykole, Kronenether, Glycerin möglich. Die Konzentration der Phasentransferstoffe im Endprodukt liegt üblicherweise zwischen 0 - 30 %, vorzugsweise bei 0 - 20 % und insbesondere bei 0 - 10 %.

Das erfindungsgemäße kontinuierliche Verfahren hat gegenüber den im Stand der Technik beschriebenen Verfahren mehrere Vorteile:
Die Anlagenkapazität erhöht sich auf Grund des Wegfalls des sequenziellen Abarbeitens der einzelnen Handlungsschritte (Befüllen, Reaktion, Methanol Abtrennung, Entleeren, Heizen, Kühlen).

Somit ist der erfindungsgemäße Prozess leichter automatisierbar und kann mit reduziertem Personalbedarf betrieben werden. Außerdem erhöht sich die Raum-Zeit-Ausbeute und die Reaktionszeiten verkürzen sich, was zu einer engeren Verweilzeitverteilung führt im Gegensatz zum Batch-Prozess.
Ferner ist die Produktqualität besser reproduzierbar und weniger schwankend, was sich insbesondere in der hohen Reinheit der erhaltenen Produkte zeigt.
So weisen die erfindungsgemäß erhaltenen Verbindungen der Formel (1) Gardner Farbzahlen von 0 bis 3, vorzugsweise von 0,1 bis 2,5, insbesondere von 0,3 bis 2,0 auf, die damit wesentlich verbessert gegenüber den im Batchverfahren erhaltenen Produkte sind, wie dies auch die nachfolgenden Beispiele zeigen. Auch der Anteil an cyclischen Nebenkomponenten fällt mit einem Anteil von < 0,05 Gew.-%, vorzugsweise < 0,03 Gew.-%, insbesondere < 0,02 Gew.-% wesentlich geringer aus als bei den bekannten Verfahren. Dadurch können die erhaltenen Produkte ohne weitere Aufreinigung auch in Anwendungen mit hohen Anforderungen (z. B. Personal Care) verwendet werden.

### Beispiele

### Beispiel 1: Cocoylglucamid aus wässriger N-Methylglucamin-Lösung und Kokosöl

Aus wässriger N-Methylglucamin-Lösung mit Natriumhydroxid wird über einen kontinuierlich, bei 145 °C, betriebenen Dünnschichtverdampfer 135 °C warme N-Methylglucamin-Schmelze hergestellt. Dies Schmelze und eine 40 °C warme Kokosölschmelze (Gustavheess (Materialnummer: 204403)) werden über einen statischen Mischer bei 130 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 35 Minuten und 11 Minuten im Rohrreaktor. Die Temperatur im Rührbehälter beträgt 130 °C, die Temperatur im Rohrreaktor beträgt 100 °C. Am Ende des Rohrreaktors kann das fertige Produkt ohne weitere Aufarbeitung abgefüllt werden.

### Beispiel 2: Cocoylglucamid aus wässriger N-Methylglucamin-Lösung und Kokosöl unter Verwendung von einem Phasentransferstoff

Aus wässriger N-Methylglucamin-Lösung mit Natriumhydroxid wird über einen kontinuierlich, bei 145 °C, betriebenen Dünnschichtverdampfer eine 135 °C warme N-Methylglucamin-Schmelze hergestellt. Die Schmelze, Propylenglycol und eine 40 °C warme Kokosölschmelze (Gustavheess (Matehainummer: 204403)) werden über einen statischen Mischer bei 130 °C vermischt. Die Mischung wird in einem kontinuierlichen Rührreaktor gepuffert und darauffolgend in einem Rohrreaktor zur Reaktion gebracht. Die Verweilzelt beträgt im Rührreaktor 25 Minuten und 8 Minuten im Rohrreaktor. Die Temperatur im Rührbehälter beträgt 100 °C, die Temperatur im Rohrreaktor beträgt 95 °C. Am Ende des Rohrreaktors kann das fertige Produkt ohne weitere Aufarbeitung abgefüllt werden.

### Beispiel 3: Oleylglucamid aus wässriger N-Methylglucamin-Lösung und Sonnenblumenöl

Aus wässriger N-Methylglucamin-Lösung mit Natriumhydroxid wird über eine kontinuierliche, bei 135 °C, betriebene zweistufige Rührkesselkaskade getrocknete N-Methylglucamin-Schmelze hergestellt. Diese Schmelze mit Zusatz von Propylenglycol und 80 °C warmes Sonnenblumenöl (Cargill (Agripur AP88, Materialnummer: 233301)) werden über einen statischen Mischer bei 120 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 55 Minuten und 21 Minuten im Rohrreaktor. Die Temperatur im Rührbehälter beträgt 110 °C, die Temperatur im Rohrreaktor beträgt 100 °C. Am Ende des Rohrreaktors kann das fertige Produkt ohne weitere Aufarbeitung abgefüllt werden.

### Beispiel 4: Octanoyl/Decanoylglucamid aus wässriger N-Methylglucamin-Lösung und Octanoyl/Decanoylmethylester unter Verwendung von einem Phasentransferstoff

Aus wässriger N-Methylglucamin-Lösung mit Natriumhydroxid wird über einen kontinuierlich, bei 145 °C, betriebenen Dünnschichtverdampfer eine 135 °C warme N-Methylglucamin-Schmelze hergestellt. Diese 135 °C warme N-Methylglucamin-Schmelze und Octanoyl/Decanoylmethylester werden unter Zusatz von Propylenglycol über einen statischen Mischer bei 120 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 10 Minuten und 3 Minuten im Rohrreaktor. Die Temperatur im Rührbehälter beträgt 85 °C, die Temperatur im Rohrreaktor beträgt 75 °C. Bei der Reaktion entsteht Methanol welches über einem kontinuierlich betriebenen Dünnschichtverdampfer, welcher am Rohrreaktorausgang angebracht ist, bei 120 °C abgetrennt wird.

### Beispiel 5: Octanoyl/Decanoylglucamid aus wässriger N-Methylglucamin-Lösung und Octanoyl/Decanoylmethylester

Aus wässriger N-Methylglucamin-Lösung mit Natriumhydroxid wird über einen kontinuierlich, bei 145 °C, betriebenen Dünnschichtverdampfer eine 135 °C warme N-Methylglucamin-Schmelze hergestellt. Diese 135 °C warme N-Methylglucamin-Schmelze und Octanoyl/Decanoylmethylester werden über einen statischen Mischer bei 120 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 17 Minuten und 4 Minuten im Rohrreaktor. Die Temperatur im Rührbehälter beträgt 95 °C, die Temperatur im Rohrreaktor beträgt 85 °C. Bei der Reaktion entsteht Methanol welches über einem kontinuierlich betriebenen Kurzwegverdampfer, welcher am Rohrreaktorausgang angebracht ist, bei 135 °C abgetrennt wird.

### Beispiel 6: n-Lauroyl-/n-Myristoyl-N-methyl-N-glucamid aus wässriger N-Methylglucamin-Lösung und Laurin/Myristinsäuremethylester unter Verwendung von einem Phasentransferstoff

Aus wässriger N-Methylglucamin-Lösung mit Natriumhydroxid wird über einen kontinuierlich, bei 145 °C, betriebenen Dünnschichtverdampfer eine 135 °C warme N-Methylglucamin-Schmelze hergestellt. Diese 135 °C warme N-Methylglucamin-Schmelze und

Laurin/Myristinsäuremethylester werden unter Zusatz von Ethanol als Phasentransferstoff über einen statischen Mischer bei 120 °C vermischt. Die Mischung wird in einem Rohrreaktor zur Reaktion gebracht. Die Verweilzeit beträgt 45 Minuten im Rohrreaktor. Die Temperatur im Rohrreaktor beträgt 155 °C. Bei der Reaktion entsteht Methanol welches über eine kontinuierlich betriebene Kolonne, welche am Rohrreaktorausgang angebracht ist, bei 135 °C abgetrennt wird.

### Beispiel 7: n-Lauroyl-/n-Myristoyl-N-methyl-N-glucamid aus wässriger N-Methylglucamin-Lösung und Laurin/Myristinsäuremethylester

Aus wässriger N-Methylglucamin-Lösung mit Natriumhydroxid wird über einen kontinuierlich, bei 140 °C, betriebenen Kurzwegverdampfer eine 130 °C warme N-Methylglucamin-Schmelze hergestellt. Diese 130 °C warme N-Methylglucamin-Schmelze und Laurin/Myristinsäuremethylester werden über einen statischen Mischer bei 130 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 33 Minuten und 13 Minuten im Rohrreaktor. Die Temperatur im Rührbehälter beträgt 100 °C, die Temperatur im Rohrreaktor beträgt 95 °C. Bei der Reaktion entsteht Methanol welches über einem kontinuierlich betriebenen Kurzwegverdampfer, welcher am Rohrreaktorausgang angebracht ist, bei 145 °C abgetrennt wird.

### Beispiel 8: Hexadecanoyl-/Octadecanoyl-N-methyl-N-glucamid aus wässriger N-Methylglucamin-Lösung und Hexadecanoyl-/Octadecanoylmethylester unter Verwendung von einem Phasentransferstoff

Aus wässriger N-Methylglucamin-Lösung mit Kaliumhydroxid wird über einen kontinuierlich, bei 145 °C, betriebenen Dünnschichtverdampfer eine 135 °C warme N-Methylglucamin-Schmelze hergestellt. Diese 135 °C warme N-Methylglucamin-Schmelze und Hexadecanoyl-/Octadecanoylmethylester werden unter Zusatz von Hexadecanoyl-/Octadecanoyl-N-methyl-N-glucamid als Phasentransferstoff über einen statischen Mischer bei 120 °C vermischt. Die Mischung wird in einem Rohrreaktor zur Reaktion gebracht. Die Verweilzeit beträgt 28 Minuten im Rohrreaktor. Die Temperatur im Rohrreaktor beträgt 105 °C. Bei der Reaktion entsteht Methanol welches über einem kontinuierlich betriebenen Dünnschichtverdampfer, welcher am Rohrreaktorausgang angebracht ist, bei 135 °C abgetrennt wird.

### Beispiel 9: Hexadecanoyl-/Octadecanoyl-N-methyl-N-glucamid aus wässriger N-Methylglucamin-Lösung und Hexadecanoyl-/Octadecanoylmethylester

Aus wässriger N-Methylglucamin-Lösung mit Natriumhydroxid wird über einen kontinuierlich, bei 145 °C, betriebenen Dünnschichtverdampfer eine 140 °C warme N-Methylglucamin-Schmelze hergestellt. Diese 140 °C warme N-Methylglucamin-Schmelze und Hexadecanoyl-/Octadecanoylmethylester werden unter Zusatz von Propylenglycol als Phasentransferstoff über einen statischen Mischer bei 130 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 17 Minuten und 5 Minuten im Rohrreaktor. Die Temperatur im Rührbehälter beträgt 95 °C, die Temperatur im Rohrreaktor beträgt 85 °C. Bei der Reaktion entsteht Methanol welches über einem kontinuierlich betriebenen Dünnschichtverdampfer, welcher am Rohrreaktorausgang angebracht ist, bei 135 °C abgetrennt wird.

### Beispiel 10: n-Dodecanoyl/ n-Docosanoyl-N-methyl-N-glucamid aus wässriger N-Methylglucamin-Lösung und n-Dodecanoyl/ n-Docosanoylmethylester

Aus wässriger N-Methylglucamin-Lösung mit Natriumhydroxid wird über einen kontinuierlich, bei 145 °C, betriebenen Dünnschichtverdampfer eine 135 °C warme N-Methylglucamin-Schmelze hergestellt. Diese 135 °C warme N-Methylglucamin-Schmelze wird unter Zusatz von Propylenglycol und n-Dodecanoyl/ n-Docosanoylmethylester über einen statischen Mischer bei 120 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 20 Minuten und 9 Minuten im Rohrreaktor. Die Temperatur im Rührbehälter beträgt 88 °C, die Temperatur im Rohrreaktor beträgt 78 °C. Bei der Reaktion entsteht Methanol welches über einem kontinuierlich betriebenen Dünnschichtverdampfer, welcher am Rohrreaktorausgang angebracht ist, bei 135 °C abgetrennt wird.

### Beispiel 11: n-Dodecanoyl-N-ethyl-N-glucamid aus wässriger N-Ethylglucamin-Lösung und n-Dodecanoylmethylester

Aus wässriger N-Ethylglucamin-Lösung mit Natriumhydroxid wird über einen kontinuierlich, bei 155 °C, betriebenen Dünnschichtverdampfer eine 145 °C warme N-Ethylglucamin-Schmelze hergestellt. Diese 145 °C warme N-Ethylglucamin-Schmelze und n-Dodecanoylmethylester werden unter Zusatz von Propylenglycol über einen statischen Mischer bei 120 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 44 Minuten und 28 Minuten im Rohrreaktor. Die Temperatur im Rührbehälter beträgt 105 °C, die Temperatur im Rohrreaktor beträgt 95 °C. Bei der Reaktion entsteht Methanol welches über einem kontinuierlich betriebenen Dünnschichtverdampfer, welcher am Rohrreaktorausgang angebracht ist, bei 135 °C abgetrennt wird.

### Beispiel 12: n-Dodecanoyl-N-octyl-N-glucamid aus wässriger N-Octylglucamin-Lösung und n-Dodecanoylmethylester

Aus wässriger N-Octylglucamin-Lösung mit Natriumhydroxid wird über einen kontinuierlich, bei 155 °C, betriebenen Dünnschichtverdampfer eine 145 °C warme N-Octylglucamin-Schmelze hergestellt. Diese 145 °C warme N-Octylglucamin-Schmelze und n-Dodecanoylmethylester werden unter Zusatz von Propylenglycol über einen statischen Mischer bei 120 °C vermischt. Die Mischung wird in einem kontinuierlichen Rührreaktor gepuffert und darauffolgend in einem Rohrreaktor zur Reaktion gebracht. Die Verweilzeit beträgt im Rührreaktor 44 Minuten und 28 Minuten im Rohrreaktor. Die Temperatur im Rührbehälter beträgt 105 °C, die Temperatur im Rohrreaktor beträgt 95 °C. Bei der Reaktion entsteht Methanol welches über einem kontinuierlich betriebenen Dünnschichtverdampfer, welcher am Rohrreaktorausgang angebracht ist, bei 135 °C abgetrennt wird.

### Beispiel 13: Batchansatz: Octanoyl/Decanoylglucamid aus wässriger N-Methylglucamin-Lösung und Octanoyl/Decanoylmethylester

Eine wässrige N-Methylglucamin-Lösung mit Natriumhydroxid wird bei 130 - 135 °C und einem Druck von 25 - 30 mbar bis zu einem Wassergehalt von unter 1 % entwässert. Die N-Methylglucamin-Schmelze wird mit Propylenglycol versetzt. Dann wird n-Octanoyl/Decanoylmethylester bei 120 °C zudosiert. Die Mischung wird in einem absatzweise betriebenen Rührreaktor unter Rückflussbedingungen zur Reaktion gebracht. Darauffolgend wird das entstandene Methanol bei einem Druck zwischen 25 mbar und 1 bar abdestilliert und die Nachreaktion bei 75 - 85 °C durchgeführt. Die Verweilzeit der gesamten Reaktion beträgt in Abhängigkeit der Ansatzgröße zwischen 3 und 8 h.

Zu den Beispielen 1 bis 13:
Beispiele 1 bis 5, 10 und 13 werden im Anschluss mit Wasser, Zitronensäure und einem Konservierungsmittel formuliert. Beispiele 6 bis 7 werden im Anschluss mit Wasser, Zitronensäure und Ethanol formuliert. Beispiele 8 und 9 werden mit Cetearylalkohol formuliert.

Im Vergleich zu einem Versuch im absatzweisen Betrieb konnte eine deutlich verbesserte Farbe des Produktes beobachtet werden. Im absatzweise betriebenen Versuch lag die Gardner Farbzahl (nach EN 1557) oberhalb 3,5. Das Produkt aus dem kontinuierlichen Betrieb weist eine Gardner-Farbzahl unter 3,5 auf. Bei der Nebenkomponente (zyklisches Glucamid) wurde ein Anteil von weniger als 0,1 Gew.-% beobachtet. Im Batchprozess liegt der Wert bei über 0,1 Gew.-%. Die APHA Farbzahlen wurden nach EN 1557 gemessen.

Tabellen zu den Prozessbedingungen der Beispiele 1 bis 12:

### Temperaturen in °C:

| Beispiel | Mischen Rohstoffe | Trocknen wässriger Lösung | Mischen Schmelze | Rührreaktor | Rohrreaktor | Entfernung Koppelprodukte |
|---|---|---|---|---|---|---|
| 1 | 70 | 145 | 130 | 130 | 100 | --- |
| 2 | 70 | 145 | 130 | 100 | 95 | --- |
| 3 | 40 | 135 | 120 | 110 | 100 | --- |
| 4 | 40 | 145 | 120 | 85 | 75 | 120 |
| 5 | 80 | 145 | 120 | 95 | 85 | 135 |
| 6 | 80 | 145 | 120 | - | 155 | 135 |
| 7 | 70 | 140 | 130 | 100 | 95 | 145 |
| 8 | 70 | 145 | 120 | - | 105 | 135 |
| 9 | 70 | 145 | 130 | 95 | 85 | 135 |
| 10 | 70 | 135 | 120 | 88 | 78 | 135 |
| 11 | 70 | 155 | 120 | 105 | 95 | 135 |
| 12 | 70 | 155 | 120 | 105 | 95 | 135 |

### Drücke in bar (abs):

| Beispiel | Mischen Rohstoffe | Trocknen wässriger Lösung | Mischen Schmelze | Rührreaktor | Rohrreaktor | Entfernung Koppelprodukte |
|---|---|---|---|---|---|---|
| 1 | 1 | 0,1 | 5 | 1 | 15 | --- |
| 2 | 1 | 0,1 | 5 | 1 | 13 | --- |
| 3 | 2 | 0,1 | 3 | 1 | 15 | --- |
| 4 | 1 | 0,1 | 3 | 1 | 7 | 0,01 |
| 5 | 1 | 0,1 | 3 | 1 | 10 | 0,05 |
| 6 | 1 | 0,1 | 3 | --- | 53 | 0,05 |
| 7 | 1 | 0,1 | 5 | 1 | 13 | 0,1 |
| 8 | 1 | 0,1 | 3 | --- | 17 | 0,05 |
| 9 | 1 | 0,1 | 5 | 1 | 10 | 0,05 |
| 10 | 1 | 0,1 | 3 | 1 | 8 | 0,03 |
| 11 | 1 | 0,2 | 6 | 1 | 13 | 0,03 |
| 12 | 1 | 0,2 | 6 | 1 | 14 | 0,03 |

### Mengenströme in kg/h:

| Beispiel | NMG/ NEG/ NOG* (wasserfrei) | NaOH / KOH | Phasentransferstoff | Ester / Triglycerid | Wasser | Zitronensäure | Ethanol / Cetearylalkohol |
|---|---|---|---|---|---|---|---|
| 1 | 2 | 0,03 | 0 | 2,4 | 5,1 | 0,04 | 0 |
| 2 | 2 | 0,03 | 0,5 | 2,4 | 5,1 | 0,04 | 0 |
| 3 | 2,9 | 0,05 | 1,9 | 4,5 | 0 | 0 | 0 |
| 4 | 2,9 | 0,04 | 0,6 | 2,7 | 4,4 | 0,06 | 0 |
| 5 | 2,9 | 0,04 | 0 | 2,7 | 4,4 | 0,06 | 0 |
| 6 | 3 | 0,05 | 0,7 | 3,6 | 2,2 | 0,12 | 1 |
| 7 | 3 | 0,05 | 0 | 3,6 | 2,2 | 0,12 | 1 |
| 8 | 2 | 0,06 | 1,5 | 2,6 | 0 | 0 | 5 |
| 9 | 2 | 0,03 | 1 | 2,6 | 0 | 0 | 5 |
| 10 | 1,7 | 0,03 | 0,48 | 2,1 | 6 | 0,02 | 0 |
| 11 | 2 | 0,03 | 0,5 | 2,5 | 0 | 0 | 0 |
| 12 | 2,5 | 0,05 | 0,5 | 2,5 | 0 | 0 | 0 |

| | | | | | | | |
|---|---|---|---|---|---|---|---|
| * NMG = N-Methylglucamin NEG = N-Ethylglucamin NOG = N-Octylglucamin | | | | | | | |

### Mengenströme in Gew.-%

| Beispiel | NMG/ NEG / NOG* (wasserfrei) | NaOH/ KOH | Phasentransferstoff | Ester / Triglycerid | Wasser | Zitronensäure | Ethanol / Cetearylalkohol |
|---|---|---|---|---|---|---|---|
| 1 | 20,9 | 0,3 | 0 | 25,1 | 53,3 | 0,4 | 0 |
| 2 | 19,9 | 0,3 | 5,0 | 23,8 | 50,6 | 0,4 | 0 |
| 3 | 31,0 | 0,5 | 20,3 | 48,1 | 0,0 | 0 | 0 |
| 4 | 27,1 | 0,4 | 5,6 | 25,2 | 41,1 | 0,6 | 0 |
| 5 | 28,7 | 0,4 | 0 | 26,7 | 43,6 | 0,6 | 0 |
| 6 | 28,1 | 0,5 | 6,6 | 33,7 | 20,6 | 1,1 | 9,4 |
| 7 | 30,1 | 0,5 | 0 | 36,1 | 22,1 | 1,2 | 10,0 |
| 8 | 17,9 | 0,5 | 13,4 | 23,3 | 0 | 0 | 44,8 |
| 9 | 18,8 | 0,3 | 9,4 | 24,5 | 0 | 0 | 47,0 |
| 10 | 16,5 | 0,3 | 4,6 | 20,3 | 58,1 | 0,2 | 0 |
| 11 | 39,8 | 0,6 | 9,9 | 49,7 | 0 | 0 | 0 |
| 12 | 45,0 | 0,9 | 9,0 | 45,0 | 0 | 0 | 0 |

| | | | | | | | |
|---|---|---|---|---|---|---|---|
| * NMG = N-Methylglucamin NEG = N-Ethylglucamin NOG = N-Octylglucamin | | | | | | | |

### Massenanteile für Batchversuch (in Gew.-%)

| Beispiel | NMG (60 % in Wasser) | NaOH / KOH | Phasentransferstoff | Ester | Wasser | Zitronensäure |
|---|---|---|---|---|---|---|
| 13 | 19,8 | 0,9 | 5,7 | 27,3 | 45,7 | 0,6 |

### Ergebnisse:

| Beispiel | Umsatz (%) bezogen auf NMG/NEG/NOG | Farbzahl Gardner | Farbzahl APHA | Methanol (Gew.-%) | Cyclische Komponenten (Gew.-%) |
|---|---|---|---|---|---|
| 1 | 96 | 0,6 | 125 | 0 | <0,01 |
| 2 | 98 | 0,6 | 125 | 0 | <0,01 |
| 3 | 98 | 0,4 | 83 | 0 | <0,01 |
| 4 | 94 | 1,7 | 354 | 0,3 | <0,01 |
| 5 | 92 | 0,7 | 146 | 0,1 | <0,01 |
| 6 | 96 | 1,2 | 250 | 0,1 | <0,01 |
| 7 | 95 | 0,3 | 63 | <0,1 | <0,01 |
| 8 | 93 | 0,6 | 125 | 0,2 | <0,01 |
| 9 | 95 | 0,7 | 146 | 0,2 | <0,01 |
| 10 | 97 | 0,4 | 83 | 0,1 | <0,01 |
| 11 | 88 | 1,8 | 375 | 0,2 | <0,02 |
| 12 | 84 | 1,9 | 380 | 0,2 | <0,02 |
| 13 | 90 | 3,6 | 500 | 0,4 | 0,1 |

| | | | | | |
|---|---|---|---|---|---|
| * NMG = N-Methylglucamin NEG = N-Ethylglucamin NOG = N-Octylglucamin | | | | | |

## Patentansprüche

1. Kontinuierliches Verfahren zur Herstellung eines Tensides, enthaltend eine Verbindung der Formel (1), wobei R² ein Fettsäurealkylrest ist und R¹ ein geradkettiger oder verzweigter C₁ bis C₁₂ Kohlenwasserstoffrest, und x im Bereich von 1 bis 15 liegt 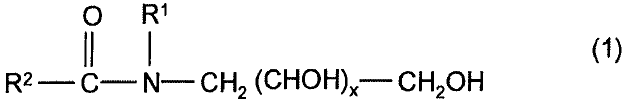 durch Umsetzung von Fettsäurealkylestern oder Fettsäuretriglyceriden mit einer N- n-alkylierten Polyhydroxyverbindung in Anwesenheit eines Alkalikatalysators oder eines Katalysators ausgewählt aus Hydroxiden oder Alkoholaten der 2. und 4. Nebengruppe des Periodensystems bei einer Temperatur im Bereich von 40 bis 300 °C, wobei das Verfahren die folgenden Schritte umfasst:
1. Mischen der wässrigen Rohstofflösung enthaltend eines oder mehrere N-Alkylglucamine mit dem Katalysator;
2. Trocknung der wässrigen Lösung, wobei der Wassergehalt nach der Trocknung im Bereich von 0 - 5 Gew.-%, liegt;
3. Erhitzen der getrockneten Mischung auf eine Temperatur im Bereich von 60 bis 180 °C und Mischen der Schmelze mit Fettsäurealkylestern oder Triglyceriden;
4. gegebenenfalls Zwischenspeichern und Mischen des Reaktionsgemisches in einem kontinuierlich betriebenen Rührkessel;
5. Reaktion im Rohrreaktor; und
6. gegebenenfalls Entfernung der Koppelprodukte.

2. Verfahren nach Anspruch 1, wobei die Fettsäurealkylester oder Fettsäuretriglyceride eine Kettenlänge von C₄ bis C₅₀ aufweisen.

3. Verfahren nach Anspruch 1 oder 2, wobei C₅ bis
C₂₆ N-Alkylglucamine verwendet werden.

4. Verfahren nach einem oder mehreren der Ansprüche 1 bis 3, wobei die Konzentration der N-Alkylglucamine in der wässrigen Lösung im ersten Verfahrensschritt im Bereich von 30 - 90 Gew.-% liegt.

5. Verfahren nach einem oder mehreren der Ansprüche 1 bis 4, wobei der Katalysator in einer Konzentration von 0,01 - 5 Gew.-% eingesetzt wird.

6. Verfahren nach einem oder mehreren der Ansprüche 1 bis 5, wobei das molare Verhältnis N-Alkylglucamin : Fettsäurealkylester 1:0,85 bis 1:1,2 oder das molare Verhältnis N-Alkylglycamin : Triglycerin 0,25:1 bis 0,45:1, beträgt.

7. Verfahren nach einem oder mehreren der Ansprüche 1 bis 6, wobei das Mischen der Glucamin Rohstofflösung im ersten Schritt bei einer Temperatur im Bereich von 40 - 120 °C erfolgt.

8. Verfahren nach einem oder mehreren der Ansprüche 1 bis 7, wobei die Reaktion der Komponenten im Rohrreaktor bei einer Temperatur im Bereich von 60 - 300 °C und einem Druck im Bereich von 0 bis 200 bar erfolgt.

9. Verfahren nach einem oder mehreren der Ansprüche 1 bis 8, wobei die Temperaturdifferenz zwischen Reaktorwand und Kernströmung in den jeweiligen Reaktoren < 20 °C beträgt.

10. Verfahren nach einem oder mehreren der Ansprüche 1 bis 9, wobei Phasentransferstoffe in einem oder mehreren der ersten drei Verfahrensschritte zur Reaktionsmischung zugegeben werden.

11. Verfahren nach Anspruch 10, wobei als Phasentransferstoffe Wasser, Alkohole, Glykole, N-Alkylglucamide, Polyalkylglykole, Kronenether und/oder Glycerin eingesetzt werden.

12. Verfahren nach einem oder mehreren der Ansprüche 1 bis 11, wobei die Polyhydroxyfettsäureamide der Formel (1) eine (Gardner)-Farbzahl im Bereich von 0 bis 3 aufweisen.

13. Verfahren nach einem oder mehreren der Ansprüche 1 bis 12, wobei der Anteil an cyclischen Nebenprodukten in den erhaltenen Polyhydroxyfettsäureamiden der Formel (1) < 0,05 Gew.-% ist.

14. Verfahren nach einem oder mehreren der Ansprüche 1 bis 13, wobei der Anteil der Verbindung (1) im Tensid im Bereich von 15 bis 100 Gew.-% liegt.

15. Verfahren nach einem oder mehreren der Ansprüche 1 bis 14, wobei die erhaltenen Verbindungen der Formel (1) in einem abschließenden Verfahrensschritt mit Lösungsmitteln oder Lösungsmittelgemischen, die optional weitere Zusatzstoffe enthalten können abgemischt werden.

## Claims

1. Continuous process for preparing a surfactant comprising a compound of the formula (1) in which R² is a fatty acid alkyl radical and R¹ is a straight-chain or branched C₁ to C₁₂ hydrocarbyl radical and x is in the range from 1 to 15 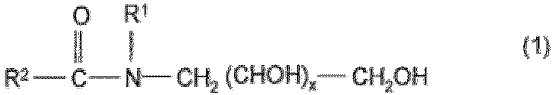 by reacting fatty acid alkyl esters or fatty acid triglycerides with an N-n-alkylated polyhydroxy compound in the presence of an alkali metal catalyst or of a catalyst selected from hydroxides or alkoxides of transition groups 2 and 4 of the Periodic Table at a temperature in the range from 40 to 300°C, wherein the process comprises the following steps:
1. mixing the aqueous raw material solution comprising one or more N-alkylglucamines with the catalyst;
2. drying the aqueous solution, the water content after drying being in the range of 0-5 wt%;
3. heating the dried mixture to a temperature in the range from 60 to 180°C and mixing the melt with fatty acid alkyl esters or triglycerides;
4. optionally intermediately storing and mixing the reaction mixture in a continuously operated stirred tank;
5. carrying out reaction in a tube reactor; and
6. optionally removing the coproducts.

2. Process according to Claim 1, wherein the fatty acid alkyl esters or fatty acid triglycerides have a chain length of C₄ to C₅₀.

3. Process according to Claim 1 or 2, wherein C₅ to C₂₆ N-alkylglucamines are used.

4. Process according to one or more of Claims 1 to 3, wherein the concentration of the N-alkylglucamines in the aqueous solution in the first process step is in the range from 30-90 wt%.

5. Process according to one or more of Claims 1 to 4, wherein the catalyst is used in a concentration of 0.01-5 wt%.

6. Process according to one or more of Claims 1 to 5, wherein the molar N-alkylglucamine : fatty acid alkyl ester ratio is 1:0.85 to 1:1.2 or the molar N-alkylglycamine : triglycerol ratio is 0.25:1 to 0.45:1.

7. Process according to one or more of Claims 1 to 6, wherein the mixing of the glucamine raw material solution in the first step takes place at a temperature in the range of 40-120°C.

8. Process according to one or more of Claims 1 to 7, wherein the reacting of the components in a tube reactor takes place at a temperature in the range of 60-300°C under a pressure in the range from 0 to 200 bar.

9. Process according to one or more of Claims 1 to 8, wherein the temperature difference between reactor wall and core flow in the respective reactors is < 20°C.

10. Process according to one or more of Claims 1 to 9, wherein phase transfer substances are added to the reaction mixture in one or more of the first three process steps.

11. Process according to Claim 10, wherein phase transfer substances used comprise water, alcohols, glycols, N-alkylglucamides, polyalkylglycols, crown ethers and/or glycerol.

12. Process according to one or more of Claims 1 to 11, wherein the polyhydroxy-fatty acid amides of the formula (1) have a (Gardner) color number in the range from 0 to 3.

13. Process according to one or more of Claims 1 to 12, wherein the fraction of cyclic byproducts in the resulting polyhydroxy-fatty acid amides of the formula (1) is < 0.05 wt%.

14. Process according to one or more of Claims 1 to 13, wherein the fraction of the compound (1) in the surfactant is in the range from 15 to 100 wt%.

15. Process according to one or more of Claims 1 to 14, wherein the compounds of the formula (1) that are obtained are blended in a concluding process step with solvents or solvent mixtures which may optionally comprise further additives.

## Revendications

1. Procédé continu pour la fabrication d'un tensioactif, contenant un composé de la formule (1), dans laquelle R² est un radical alkyle d'acide gras et R¹ est un radical hydrocarboné en C₁ à C₁₂ linéaire ou ramifié, et x se situe dans la plage allant de 1 à 15, 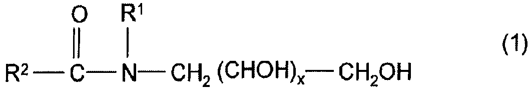 par mise en réaction d'esters alkyliques d'acides gras ou de triglycérides d'acides gras avec un composé polyhydroxy N-n-alkylé en présence d'un catalyseur alcalin ou d'un catalyseur choisi parmi les hydroxydes ou les alcoolates des groupes de transition 2 et 4 du tableau périodique à une température dans la plage allant de 40 à 300 °C, le procédé comprenant les étapes suivantes :
1. le mélange de la solution aqueuse de matières premières contenant une ou plusieurs N-alkylglucamines avec le catalyseur ;
2. le séchage de la solution aqueuse, la teneur en eau après le séchage se situant dans la plage allant de 0 à 5 % en poids ;
3. le chauffage du mélange séché à une température dans la plage allant de 60 à 180 °C et le mélange de la masse fondue avec des esters alkyliques d'acides gras ou des triglycérides ;
4. éventuellement l'entreposage et le mélange du mélange réactionnel dans une cuve agitée exploitée en continu ;
5. la réaction dans le réacteur tubulaire ; et
6. éventuellement l'élimination des sous-produits.

2. Procédé selon la revendication 1, dans lequel les esters alkyliques d'acides gras ou les triglycérides d'acides gras présentent une longueur de chaîne de C₄ à C₅₀.

3. Procédé selon la revendication 1 ou 2, dans lequel des N-alkylglucamines en C₅ à C₂₆ sont utilisées.

4. Procédé selon une ou plusieurs des revendications 1 à 3, dans lequel la concentration des N-alkylglucamines dans la solution aqueuse dans la première étape de procédé se situe dans la plage allant de 30 à 90 % en poids.

5. Procédé selon une ou plusieurs des revendications 1 à 4, dans lequel le catalyseur est utilisé en une concentration de 0,01 à 5 % en poids.

6. Procédé selon une ou plusieurs des revendications 1 à 5, dans lequel le rapport molaire N-alkylglucamine: esters alkyliques d'acides gras est de 1:0,85 à 1:1,2, ou le rapport molaire N-alkylglucamine:triglycérine est de 0,25:1 à 0,45:1.

7. Procédé selon une ou plusieurs des revendications 1 à 6, dans lequel le mélange de la solution de matières premières de glucamine dans la première étape a lieu à une température dans la plage allant de 40 à 120 °C.

8. Procédé selon une ou plusieurs des revendications 1 à 7, dans lequel la réaction des composants dans le réacteur tubulaire a lieu à une température dans la plage allant de 60 à 300 °C et une pression dans la plage allant de 0 à 200 bar.

9. Procédé selon une ou plusieurs des revendications 1 à 8, dans lequel la différence de température entre la paroi du réacteur et l'écoulement central dans les réacteurs respectifs est < 20 °C.

10. Procédé selon une ou plusieurs des revendications 1 à 9, dans lequel des matières de transfert de phase sont ajoutées au mélange réactionnel dans une ou plusieurs des trois premières étapes de procédé.

11. Procédé selon la revendication 10, dans lequel de l'eau, des alcools, des glycols, des N-alkylglucamides, des polyalkylglycols, des éthers couronnes et/ou de la glycérine sont utilisés en tant que matières de transfert de phase.

12. Procédé selon une ou plusieurs des revendications 1 à 11, dans lequel les amides d'acides polyhydroxy-gras de la formule (1) présentent un indice de couleur (Gardner) dans la plage allant de 0 à 3.

13. Procédé selon une ou plusieurs des revendications 1 à 12, dans lequel la proportion de produits secondaires cycliques dans les amides d'acides polyhydroxy-gras de la formule (1) obtenus est < 0,05 % en poids.

14. Procédé selon une ou plusieurs des revendications 1 à 13, dans lequel la proportion du composé (1) dans le tensioactif se situe dans la plage allant de 15 à 100 % en poids.

15. Procédé selon une ou plusieurs des revendications 1 à 14, dans lequel les composés de la formule (1) obtenus sont mélangés dans une étape de procédé finale avec des solvants ou des mélanges de solvants, qui peuvent éventuellement contenir des additifs supplémentaires.
